# EUROPEAN PATENT SPECIFICATION

(11) **EP 4 329 908 B1**
(45) Date of publication and mention of the grant of the patent: **17.06.2026**
(21) Application number: 22725481.0
(22) Date of filing: 26.04.2022
(51) Int. Cl.: B01D 1/30, B01D 3/34, C07C 273/04, C07C 273/16

(54) **IMPROVEMENTS TO EQUIPMENT OF UREA PLANTS**
VERBESSERUNGEN AN EINER AUSRÜSTUNG EINER HARNSTOFFANLAGE
AMÉLIORATIONS APPORTÉES AUX ÉQUIPEMENTS DES USINES D'URÉE

(30) Priority: 28.04.2021 EP 21170937
(43) Date of publication of application: 06.03.2024
(73) Proprietor: CASALE SA, 6900 Lugano (CH)
(72) Inventor: BERETTI, Andrea, 22069 Rovellasca (CO) (IT); VERNIER, Maddalena, 22100 Como (CO) (IT); LANZANI, Riccardo, 20823 Lentate Sul Seveso (MB) (IT); BERTINI, Paolo, 6900 Lugano (CH)
(74) Representative: M. Zardi & Co S.A.
(86) International application number: PCT/EP2022/060995
(87) International publication number: WO 2022/229148

(56) References cited:
- JP-A- S63 119 801
- US-A- 2 819 206
- US-A- 4 113 552
- US-A- 4 486 270
- US-A1- 2005 261 530

## Description

### Field of application

The invention relates to improvements of certain equipment of a urea production plant. Particularly the invention relates to an improved design applicable to urea strippers and evaporators.

### Prior art

Urea is produced industrially by reacting ammonia and carbon dioxide under suitable urea-forming conditions, which typically include a high pressure of 100 to 200 bar and a high temperature around 200 °C. An overview of the industrial processes for the synthesis of urea can be found in Meessen, "Urea", Ullmann's Encyclopaedia of Industrial Chemistry, Wiley-VCH Verlag, 2010.

The reaction mixture that can be obtained from a synthesis reactor is an aqueous solution including urea and a significant amount of unconverted reagents, typically in the form of ammonium carbamate. In a urea stripping process, the reaction mixture is processed in a urea stripper wherein ammonium carbamate contained in the mixture is thermally decomposed to obtain a purified urea solution and a gaseous stream including ammonia and carbon dioxide removed from the mixture.

The purified urea solution still contains come carbamate and is normally sent to one or more recovery sections operating at a lower pressure to further decompose and remove the ammonium carbamate; the ammonia and carbon dioxide emerging from the stripper are normally condensed and recycled to the reactor.

The stripping process may be performed with the help of a stripping medium.

The known CO2 stripping process uses gaseous CO2 as a stripping medium. In the alternative ammonia-stripping process, the reaction mixture contains excess of ammonia so that ammonia can act as a stripping aid.

The urea stripper is normally a vertical shell-and-tube apparatus wherein the reaction mixture flows downwards inside the tubes of a tube bundle, usually in a falling-film regime. The tube bundle is heated by hot steam traversing the shell side to provide the heat for decomposition of carbamate. The stripping medium, if used, is generally fed counter-current to the solution.

The urea solution effluent from the tubes, i.e. after the stripping process, is collected in the lower part of the stripper. Particularly, the known strippers have a bottom liquid holder wherein the purified urea solution is collected. The bottom liquid holder is directly in communication with a liquid outlet port of the stripper. The ammonia and CO2 vapours on the other hand are generally withdrawn from top of the stripper.

The bottom holder is usually equipped with a vortex breaker to avoid the formation of a vortex in proximity of the outlet nozzle.

The applicant has noted that urea strippers suffer from a problem of gas carry-over. This term denotes the undesired occurrence that some process gas contained in the stripper (predominantly ammonia and/or carbon dioxide) is carried by the liquid effluent. It is desirable to maintain a good separation between the liquid phase and the gaseous phase to avoid said gas carry-over. However, the applicant has noted the bottom part of the stripper, wherein the processed liquid is collected and directed towards the liquid outlet, is particularly exposed to the risk of gas carryover.

The applicant has further noted that gas carryover may occur in the evaporators of urea plants. An evaporator is provided to remove water from the aqueous solution produced in a recovery section. Said aqueous solution contains urea, water and unavoidable impurities. By removing water, the solution is converted into a more concentrated solution or a urea melt, for example a urea melts suitable for a finishing process like prilling or granulation. The urea melt may have a concentration greater than 96 wt% of urea or greater than 98 wt% or greater than 99 wt% according to the use. For a prilling process a purity greater than 99.5 wt% may be required. The applicant has noted that the concentrated solution or urea melt may entrain some process gas, predominantly ammonia and carbon dioxide or inert gas. An example of a stripper/evaporator with a bottom liquid holder with screens and a vortex breaker is disclosed in US4113552.

### Summary of the invention

The invention addresses the technical problem of how to avoid or reduce the drawback of gas carryover in a urea stripper or urea evaporator, as above disclosed. Accordingly, the invention aims to increase the separation between the liquid effluent and process gas contained in the stripper or evaporator. This separation is also termed phase disengagement. Accordingly, the patent aims to improve the phase disengagement at the liquid outlet of the stripper or of the evaporator.

The applicant has realized that a common feature of urea strippers and evaporators is that the volume of the liquid holder (hold up volume) is comparatively small to reduce the residence time of the liquid (typically around 5 to 20 seconds) and related problems of formation of undesired by-products such as biuret. This is particularly true for large items designed for a large capacity, wherein the size of the main pressure vessel is comparatively larger. As a consequence, the liquid flow entering the holder has a high velocity and momentum which may promote the carry-over of process gas, typically of ammonia, carbon dioxide and inert gases. The idea underlying the invention is to provide the bottom of the apparatus, particularly the liquid holder, with fittings adapted to oppose this effect.

The invention is defined by the independent claims.

In accordance with the above, the invention includes the following:
a) a plurality of perforated screens horizontally arranged in the bottom liquid holder at different elevation.

The above item represents a fitting adapted to reduce the velocity of the liquid stream inside the bottom liquid holder and to limit the undesired formation of liquid-gas recirculation. More precisely the above item can break the liquid flow and reduce its velocity and momentum in the lower part of the apparatus and particularly at the liquid outlet. The applicant has found that breaking the liquid flow and reducing its velocity and momentum can greatly reduce the undesired gas carryover.

Further aspects of the invention are a urea plant including the inventive urea stripper and/or the inventive evaporator, and a method for revamping such items. Particularly the various embodiments of the invention can be applied to the provision of a new stripper or evaporator as well as to the modification of an existing stripper or of an existing evaporator.

The stripper is normally included in a high-pressure synthesis loop including a reactor and a condenser and optionally a scrubber.

The modification of an existing item is an interesting embodiment of the invention. A relatively inexpensive modification of the lower part of the apparatus may give a significant improvement in terms of performance. For example, referring to the modification of a stripper, the invention gives a better separation of phases thus improving the recycle of the gaseous phase to the condenser and the operation of the recovery section downstream, where the liquid effluent of the stripper is processed.

### Description of the invention

The above option a) of the invention, and options from b) to d) that are not part of the present invention, are now elucidated in a greater detail.

The option a) includes a plurality of perforated screens arranged at different elevation in the bottom liquid holder. The plurality of perforated screens includes disc-shaped screens and ring-shaped screens. Said screens are be stacked one above the other.

In an embodiment each of the perforated screens has a surface area substantially equal to the cross section of the liquid holder.

The plurality of perforated screens includes screens with a surface area smaller than the cross section of the liquid holder and the screens are arranged to cover different portions of the cross section. Particularly, at least two consecutive screens may be staggered or offset to provide a sort of labyrinth path for the liquid stream flowing into the liquid holder.

Option a) includes a horizontal screen proximal to a liquid outlet duct of the liquid holder. Said horizontal screen has preferably an unperforated central region adapted to act as a vortex breaker and a perforate annular region around said central region. Said screen may be spaced apart from the outlet duct by suitable supports so that the liquid flows into the outlet duct through passages between the supports. The supports may be substantially shaped like blades.

One or more perforated screen are integral with a vortex breaker installed in the liquid holder. A vortex breaker is usually provided in the bottom liquid holder of a stripper.

An option b) - that is not part of the present invention - may include a perforated cylindrical wall placed in the bottom liquid holder, so that the inside of the bottom liquid holder is divided into an annular chamber around said wall and a central chamber inside said wall. Said chambers are communication through the perforations of said wall and only the central chamber is in communication with the liquid outlet. The liquid stream (e.g. aqueous solution from a stripper) is collected predominantly in the annular chamber; from here it passes into the central chamber through the perforated wall and eventually into the liquid outlet.

In a further embodiment the option b) includes a vertically arranged perforated cylindrical wall extending downward from an unperforated horizontal screen. Said vertically arranged perforated wall may be realized with the known technique of Johnson screens or equivalent. In some embodiments it can be a slotted wall with a number of slots providing a suitable passage area.

According to yet another embodiment that is not part of the present invention, an option c) includes a closed cylindrical chamber placed in the bottom liquid holder, so that the only region of the bottom liquid holder where liquid can be collected is an annular region around said sealed chamber. Said closed chamber may be delimited by a section of tube fitted in the liquid holder.

Said closed chamber reduced the volume available to the liquid in the bottom holder, so that for a given amount (flowrate) of liquid a higher level will form in the holder. A higher liquid level improves the phase disengagement.

An option d) - that is not part of the present invention - may include a plurality of blades of a suitable shape and/or vertical plates projecting from the inner surface of a bottom domed end of the apparatus.

An option d) - that is not part of the present invention - is particularly interesting for a stripper. In operation, the solution effluent from the tubes flows over the inner surface of the domed end towards the bottom liquid holder. The provision of a suitable number of blades or vertical plates reduces the velocity and momentum of the liquid flow travelling towards the bottom holder.

The invention is applicable to all kinds of urea strippers. The urea stripper may be adapted to operate according to any of: the self-stripping process, the ammonia-stripping process, the CO2 stripping process. A stripper for the CO2 stripping process includes a CO2 inlet and a CO2 distributor in the lower part. The CO2 distributor is adapted to provide an uprising flow of gaseous CO2 in the tubes of the tube bundle, counter-current relative to the falling film of liquid.

The urea stripper is generally part of a high-pressure synthesis section or high-pressure synthesis loop. Said loop may include, in addition to the stripper, a urea synthesis reactor and a high-pressure condenser. The high-pressure condenser typically receives the gaseous stream from the stripper and a recycle carbamate solution from a recovery section. The loop may also include a high-pressure scrubber when vapours and inert gas withdrawn from top of the reactor are scrubbed with a carbamate solution.

The evaporator to which the invention is applied may be part of an evaporation section comprising more than one evaporator.

A further aspect of the invention is a method for revamping a stripper or an evaporator of a urea plant according to the claims.

A particularly interesting embodiment provides the modification or replacement of the vortex breaker. A urea stripper is normally equipped with a vortex breaker which includes unperforated screen positioned above the entrance of the liquid outlet and a suitable number (e.g. four) of lateral blade supports. The liquid flowing from above can enter the liquid outlet only by flowing laterally between the support blades as the screen prevents the liquid from falling vertically and directly into the liquid outlet.

According to the invention said vortex breaker can be modified or replaced providing a perforated horizontal annular screen around said unperforated vortex-breaker screen. As a result, a first horizontal screen is provided with an unperforated central region surrounded by a perforated annular region. In some embodiments, one or more additional perforated screens, horizontally arranged, may be placed above said first screen.

Some embodiments of the inventive method of revamping can be put into practice by a modification or replacement of the existing vortex breaker. A vortex breaker can be modified for example by adding one or more perforated screen(s) to the conventional structure of the vortex breaker, as will be elucidated below with some examples.

In another embodiment a modified vortex breaker includes an unperforated horizontal first screen and a vertically arranged annular perforated screen extending down from said first screen.

Embodiments of the revamping method based on modified vortex breakers may be of particular interest due to easy and inexpensive implementation. Particularly, the vortex breaker is relatively easy to access by opening the lower part of the pressure vessel and therefore the modification can be carried out on site in a relatively short time, which is a considerable advantage. Usually, urea strippers and evaporators have a bottom manhole so that operation inside the lower part of its vessel is possible without complication.

### Description of the figures

Fig. 1 is a sketch of a urea stripper for CO2 stripping process according to the prior art.
Fig. 2 is a detail of the bottom of the stripper of Fig. 1, according to the prior art.
Figs. 3, and from 5 to 7 illustrate embodiments that are not part of the invention.
Fig. 4 illustrates an embodiment of the invention.
Fig. 8 illustrate in detail a prior art vortex breaker of a urea stripper.
Figs. 9, 10 illustrate a modified vortex breaker according to some embodiments that are not part of the invention.
Fig. 11 illustrate a sketch of an evaporator of a urea plant.
Fig. 12 is a detail of the liquid holder of the evaporator of Fig. 11.
Fig. 13 illustrates a further embodiment of modified vortex breaker that is not part of the invention.

### Detailed description

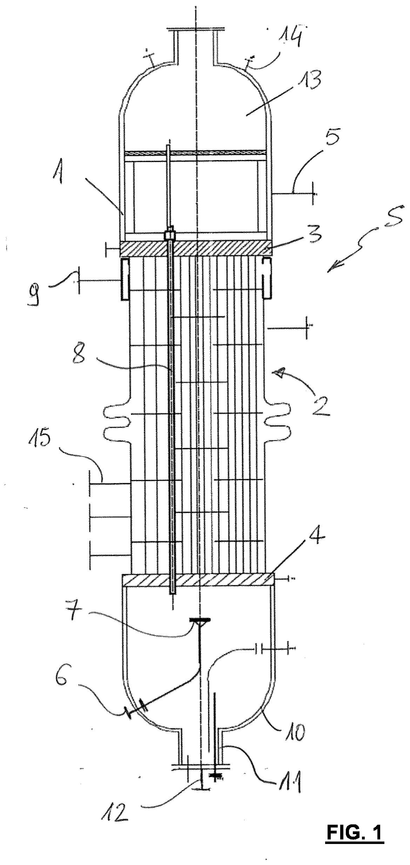

Fig. 1 illustrates a typical embodiment of a vertical shell-and-tube CO2 stripper S with the following items.
- 1: Pressure vessel
- 2: Stripping section containing a tube bundle
- 3: Upper tubesheet
- 4: Lower tubesheet
- 5: Reaction mixture inlet
- 6: Gaseous CO2 inlet
- 7: CO2 distributor
- 8: Exemplary tube of the tube bundle
- 9: Steam inlet in communication with the shell side of the section 2 around the tubes
- 10: Bottom domed end
- 11: Bottom liquid holder
- 12: Liquid outlet (urea-carbamate solution after stripping)
- 13: Upper chamber for collection of gaseous ammonia and carbon dioxide
- 14: Gas outlet, e.g. to condenser
- 15: Steam/condensate outlets

The above features of a urea stripper are known to the skilled person and need not be described in detail.

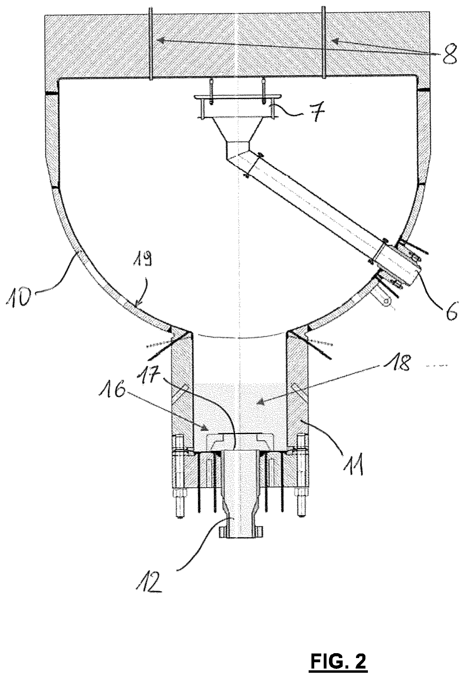

Fig. 2 illustrates a detail of the lower part of the stripper, particularly the bottom end 10, the bottom liquid holder 11 and the liquid outlet 12. Fig. 2 further illustrates a vortex breaker 16 placed right above the inlet section 17 of the liquid outlet 12. Fig. 2 further illustrates the liquid level 18 inside the liquid holder 11.

The liquid holder 11 is basically a cylinder vessel with a diameter smaller than that of the stripper, i.e. smaller than the main pressure vessel 1.

In operation, the solution effluent from the tubes 8 flows over the inner surface 19 of the domed end 10 and is collected in the liquid holder 11 forming the liquid level 18. Flowing laterally around the vortex breaker 16, the liquid finally enters the outlet 12 and leaves the stripper.

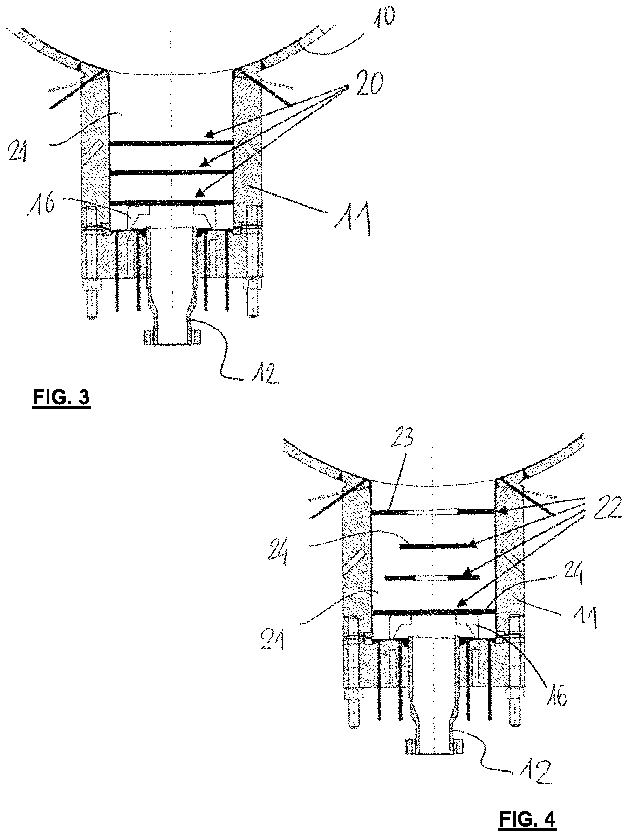

Fig. 3 illustrates a first embodiment that is not part of the invention wherein the stripper is fitted with horizontal perforated screens 20 placed in the liquid holder 11 above the vortex breaker 16. For example, Fig. 3 illustrates an embodiment with three additional screens 20. The screens 20 are placed in the chamber 21 formed by the liquid holder 11 and are preferably full-diameter screen extend over the entire cross section of the chamber 21.

Accordingly, the solution flows into the outlet 12 through the perforated screens 20 which reduce the velocity and momentum of the liquid flow and increase phase separation.

Fig. 4 illustrates an embodiment with multiple perforated screens 22 arranged to create a labyrinth path for the fluid along the chamber 21. Particularly the screens 22 include rings 23 and perforated discs 24. Preferably at least some of the screens have a diameter smaller than the inner diameter of the chamber 21. A full diameter screen 24 is also provided in Fig. 4. Said full diameter screen may coincide with the screen of the vortex breaker 16, in a preferred embodiment.

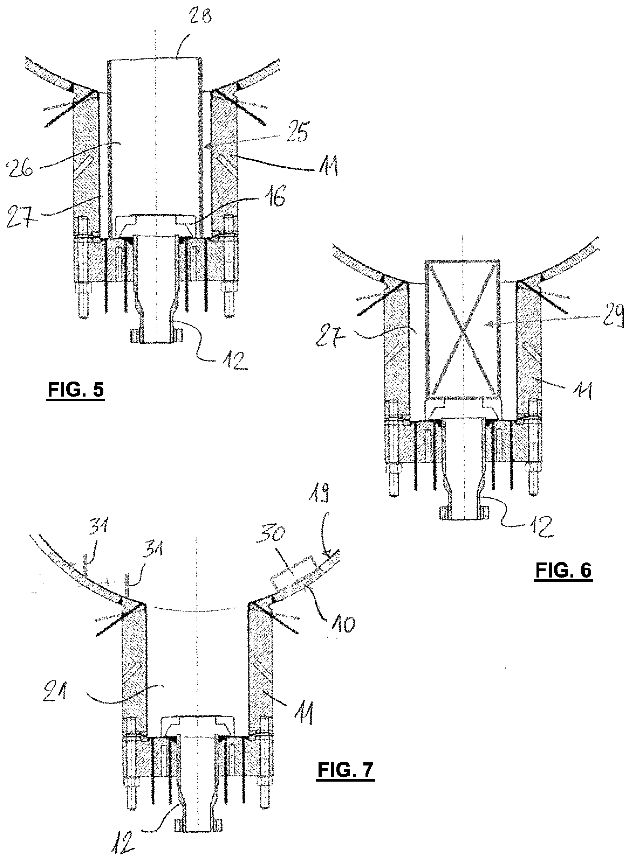

Fig. 5 illustrates an embodiment that is not part of the invention where the liquid holder 11 is internally fitted with a perforated wall 25 so that the chamber 21 is divided by the wall 25 into a central chamber 26 and an annular chamber 27. The upper section 28 of the wall 25 opens into the lower chamber of the stripper. In this embodiment the urea solution is predominantly collected in the annular chamber 27, as it flows over the surface 19 of the domed end. The solution collected in said annular chamber 27 reaches the outlet 12 through the perforated wall 25. A portion of the solution which flows directly into the central chamber 26 will flow around the vortex breaker 16.

Fig. 6 illustrates a variant that is not part of the invention wherein the liquid holder is fitted with a closed tube 29 so that only the chamber 27 is available to collect the liquid. Basically, this embodiment reduces the volume of the chamber 21 to increase the liquid level in the chamber.

Fig. 7 illustrates an embodiment that is not part of the invention with blades 30 and/or vertical plates or screens distributed over the inner face 19 of the domed end 10. These fittings slow down the urea solution before it enters the chamber 21.

The various embodiments of the invention are not mutually exclusive and can be combined. In principle, all embodiments described herein can be freely combined.

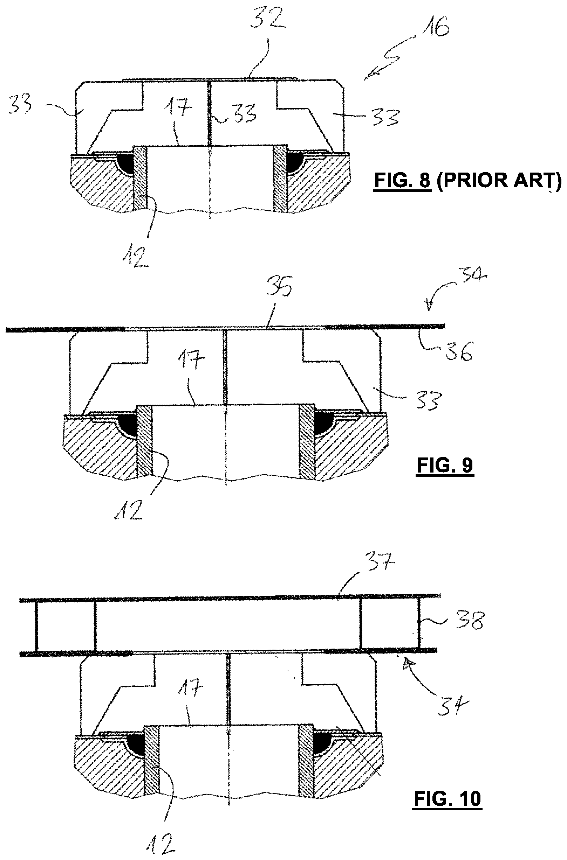

Figs. 8 to 10 relate to an interesting embodiment that is not part of the invention wherein horizontal perforated screens are integrated with the vortex breaker 16.

Fig. 8 illustrates a typical embodiment of the vortex breaker 16 including an unperforated screen 32 over the inlet section 17 of the liquid outlet 12. The screen 32 is distanced from the inlet section 17 by blade-like supports 33.

Fig. 9 illustrates an embodiment that is not part of the invention wherein the screen 32 is replaced by a larger screen 34 having an unperforated central region 35 surrounded by a perforated annular region 36.

Fig. 10 illustrates a further embodiment that is not part of the invention including, in addition to the screen 34, a second perforated screen 37 connected to the screen 34 by suitable supports 38. The second (upper) screen 37 may be perforated over the entire surface whilst the first (lower) screen 34 is only perforated in the region 35.

Figs. 9 and 10 can be obtained by means of a modification or replacement of the existing vortex breaker 16.

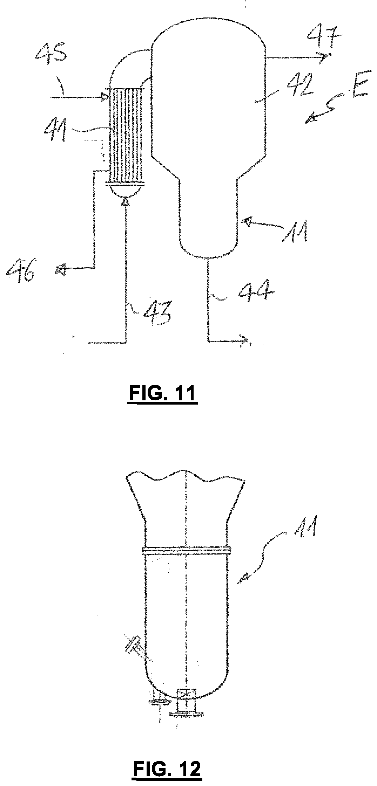

Fig. 11 discloses a vacuum evaporator E including a tube heat exchanger 41 and a pressure vessel 42. Fig. 11 illustrates a urea solution input line 43, a urea melt output line 44, a steam input line 45 and a steam/condensate output line 46, a vapour line 47. The pressure vessel 42 has a bottom liquid holder 11 for collection of the urea melt. The input line 43 may be connected to a first evaporator upstream the evaporator E.

In operation, the urea solution of line 43 traverses the tube side of the shell-and-tube exchanger 41, wherein the tubes are externally heated by steam 45 in the shell side. Water is then removed in the vessel 42 under vacuum and leaves the vessel via line 47.

The above embodiments and combinations thereof for reducing the gas carryover are applicable to the liquid holder of the evaporator E. This applies in particular to option a) as above described and to the embodiment of Fig. 4. The fittings as above described may be provided in the bottom liquid holder of the evaporator E which is illustrated in Fig. 12.

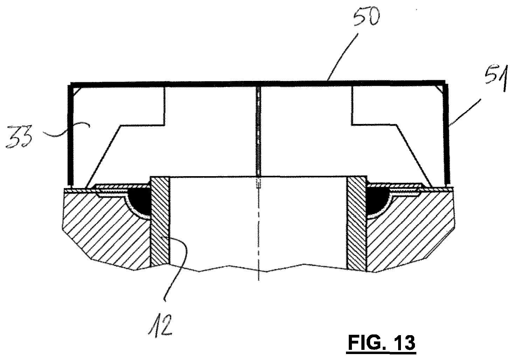

Fig. 13 illustrates a further embodiment that is not part of the invention which may be realised upon modification of a vortex breaker. In this embodiment the modified vortex breaker includes an unperforated horizontal first screen 50 and a vertically arranged annular perforated screen 51 extending down from said first screen 50. The perforated screen 51 may be realised with a slotted wall or a Johnson screen or equivalent.

## Claims

1. An apparatus of a urea production plant, wherein the apparatus is a urea stripper (S) or an evaporator (E);
wherein said urea stripper (S) is adapted to process a urea-containing reaction mixture for decomposition of unreacted ammonium carbamate contained in the mixture into a gaseous mixture of ammonia and carbon dioxide, wherein the urea stripper includes a stripping section (2) wherein the stripping process is performed and a bottom liquid holder (11) arranged to collect urea-containing solution after the stripping process;
wherein said evaporator (E) is adapted to process an aqueous urea solution to remove water and to obtain a concentrated solution or a urea melt, wherein the evaporator includes a pressure vessel operated under vacuum and a bottom liquid holder (11) arranged to collect the concentrated solution or the urea melt;
said bottom liquid holder (11) being a cylinder vessel with a diameter smaller than that of a pressure vessel of said urea stripper (S) or of said pressure vessel of the evaporator (E) and having a liquid outlet (12) at its bottom;
the urea stripper (S) or the evaporator (E) further comprises:
a plurality of perforated screens (20) horizontally arranged inside the bottom liquid holder at different elevation;
wherein said plurality of perforated screens includes disc-shaped screens (20, 24) and ring-shaped screens (23) stacked one above the other; wherein said plurality of perforated screens includes screens arranged so that at least two screens placed one above the other extend over different portions of the cross section of an internal chamber of the liquid holder;
wherein the apparatus includes that one or more horizontal screen(s) is/are integral with a vortex breaker (16) fitted inside the bottom liquid holder (11).

2. Use of the apparatus according to claim 1 as a urea stripper adapted to operate according to any of: the self-stripping process, the ammonia-stripping process, the CO2 stripping process.

3. A urea production plant including at least one apparatus according to claim 1.

4. A method for revamping a urea stripper or a vacuum evaporator of a urea production plant;
wherein the urea stripper includes a stripping section wherein the stripping process is performed and a bottom liquid holder wherein urea-containing solution is collected after the stripping process,
wherein the vacuum evaporator includes a vessel arranged to remove water from an aqueous solution of urea for the production of a concentrated solution or of a urea melt, said vessel including a bottom liquid holder where the concentrated solution or urea melt is collected; the method comprising the provision of:
a) perforated screens horizontally arranged in the bottom portion;
wherein option a) includes the provision of a plurality of perforated screens (20) arranged at different elevation in the bottom liquid holder, the plurality of perforated screens including disc-shaped screens and ring-shaped screens stacked one above the other;
wherein the option a) includes the provision of screens arranged so that at least two screens which are placed one above the other extend over different portions of cross section of an internal chamber (21) of the liquid holder;
wherein option a) includes that one or more horizontal screen(s) is/are integral with a vortex breaker (16) fitted inside the bottom liquid holder (11).

## Patentansprüche

1. Eine Vorrichtung einer Harnstoffproduktionsanlage, wobei es sich bei der Vorrichtung um einen Harnstoff-Stripper (S) oder einen Verdampfer (E) handelt; wobei der Harnstoff-Stripper (S) dazu ausgelegt ist, ein harnstoffhaltiges Reaktionsgemisch zu verarbeiten, um in dem Gemisch enthaltenes, nicht umgesetztes Ammoniumcarbamat in ein gasförmiges Gemisch aus Ammoniak und Kohlendioxid zu zersetzen, wobei der Harnstoff-Stripper einen Stripping-Abschnitt (2), in dem der Strippverfahren durchgeführt wird, und einen Bodenflüssigkeitsbehälter (11) umfasst, der so angeordnet ist, dass er die harnstoffhaltige Lösung nach dem Strippverfahren auffängt;
wobei der Verdampfer (E) dazu ausgelegt ist, eine wässrige Harnstofflösung zu verarbeiten, um Wasser zu entfernen und eine konzentrierte Lösung oder eine Harnstoffschmelze zu erhalten, wobei der Verdampfer ein unter Vakuum betriebenes Druckgefäß und einen Bodenflüssigkeitsbehälter (11) umfasst, der zum Auffangen der konzentrierten Lösung oder der Harnstoffschmelze angeordnet ist;
wobei der Bodenflüssigkeitsbehälter (11) ein zylindrischer Gefäß mit einem Durchmesser ist, der kleiner als der eines Druckgefäßes des Harnstoff-Strippers (S) oder des Druckgefäßes des Verdampfers (E) ist, und an seinem Boden einen Flüssigkeitsauslass (12) aufweist;
wobei der Harnstoff-Stripper (S) oder der Verdampfer (E) ferner umfasst:
eine Mehrzahl von perforierten Sieben (20), die horizontal in unterschiedlichen Höhen im Bodenflüssigkeitsbehälter angeordnet sind;
wobei die Mehrzahl der perforierten Siebe scheibenförmige Siebe (20, 24) und ringförmige Siebe (23) umfasst, die übereinander gestapelt sind;
wobei die Mehrzahl der perforierten Siebe Siebe umfasst, die so angeordnet sind, dass sich mindestens zwei übereinander angeordnete Siebe über verschiedene Abschnitte des Querschnitts einer Innenkammer des Flüssigkeitsbehälters erstrecken;
wobei bei der Vorrichtung ein oder mehrere horizontale Siebe fest mit einem Wirbelbrecher (16) verbunden sind, der im Bodenflüssigkeitsbehälter (11) angebracht ist.

2. Verwendung der Vorrichtung nach Anspruch 1 als Harnstoff-Stripper, der für den Betrieb nach einem der folgenden Verfahren ausgelegt ist: dem Selbststrippverfahren, dem Ammoniak-Strippverfahren oder dem CO₂-Strippverfahren.

3. Eine Harnstoffproduktionsanlage, die mindestens eine Vorrichtung nach Anspruch 1 umfasst.

4. Ein Verfahren zum Umgestalten eines Harnstoff-Strippers oder eines Vakuumverdampfers einer Harnstoffproduktionsanlage;
wobei der Harnstoff-Stripper einen Stripping-Abschnitt, in dem das Strippverfahren durchgeführt wird, und einen Bodenflüssigkeitsbehälter umfasst, in dem die harnstoffhaltige Lösung nach dem Strippverfahren gesammelt wird,
wobei der Vakuumverdampfer ein Gefäß umfasst, das so angeordnet ist, dass es Wasser aus einer wässrigen Harnstofflösung entfernt, um eine konzentrierte Lösung oder eine Harnstoffschmelze herzustellen, wobei das Gefäß einen Bodenflüssigkeitsbehälter umfasst, in dem die konzentrierte Lösung oder die Harnstoffschmelze gesammelt wird;
wobei das Verfahren die Bereitstellung umfasst von:
a) im Bodenbereich horizontal angeordneter perforierter Siebe;
wobei Option a) die Anordnung einer Mehrzahl von perforierten Sieben (20) umfasst, die in unterschiedlichen Höhen im Bodenflüssigkeitsbehälter angeordnet sind, wobei die Mehrzahl der perforierten Siebe scheibenförmige Siebe und ringförmige Siebe umfasst, die übereinander gestapelt sind;
wobei Option a) die Bereitstellung von Sieben umfasst, die so angeordnet sind, dass sich mindestens zwei Siebe, die übereinander angeordnete platziert sind, über verschiedene Querschnittsteile einer Innenkammer (21) des Flüssigkeitsbehälters erstrecken;
wobei Option a) umfasst, dass ein oder mehrere horizontale Siebe fest mit einem Wirbelbrecher (16) verbunden sind, der im Bodenflüssigkeitsbehälter (11) angebracht ist.

## Revendications

1. Appareil d'une installation de production d'urée, dans lequel l'appareil est un décapant d'urée (S) ou un évaporateur (E) ;
dans lequel ledit décapant d'urée (S) est adapté pour traiter un mélange réactionnel contenant de l'urée pour la décomposition de carbamate d'ammonium n'ayant pas réagi contenu dans le mélange en un mélange gazeux d'ammoniac et de dioxyde de carbone, dans lequel le décapant d'urée comprend une section de décapage (2) dans laquelle le processus de décapage est réalisé et un contenant de liquide inférieur (11) agencé pour collecter une solution contenant de l'urée après le processus de décapage ;
dans lequel ledit évaporateur (E) est adapté pour traiter une solution aqueuse d'urée pour éliminer l'eau et pour obtenir une solution concentrée ou une fusion d'urée, dans lequel l'évaporateur comprend un récipient sous pression fonctionnant sous vide et un contenant de liquide inférieur (11) agencé pour collecter la solution concentrée ou la fusion d'urée ;
ledit contenant de liquide inférieur (11) étant un récipient cylindrique d'un diamètre inférieur à celui d'un récipient sous pression dudit décapant d'urée (S) ou dudit récipient sous pression de l'évaporateur (E) et présentant une sortie de liquide (12) au niveau de son fond ;
le décapant d'urée (S) ou l'évaporateur (E) comporte en outre :
une pluralité de tamis perforés (20) agencés horizontalement à l'intérieur du contenant de liquide inférieur à une hauteur différente ;
dans lequel ladite pluralité de tamis perforés comprend des tamis discoïdes (20, 24) et des tamis annulaires (23) empilés les uns au-dessus des autres ;
dans lequel ladite pluralité de tamis perforés comprend des tamis agencés de sorte qu'au moins deux tamis placés l'un au-dessus de l'autre s'étendent sur différentes parties de la section transversale d'une chambre interne du contenant de liquide ;
dans lequel dans l'appareil, un ou plusieurs tamis horizontaux sont intégrés à un déflecteur antitourbillon (16) monté à l'intérieur du contenant de liquide inférieur (11).

2. Utilisation de l'appareil selon la revendication 1 en tant que décapant d'urée adapté pour fonctionner selon l'un quelconque des procédés suivants : le procédé d'autodécapage, le procédé de décapage à l'ammoniac, le procédé de décapage au CO₂.

3. Installation de production d'urée comprenant au moins un appareil selon la revendication 1.

4. Procédé de modernisation d'un décapant d'urée ou d'un évaporateur sous vide d'une installation de production d'urée ;
dans lequel le décapant d'urée comprend une section de décapage dans lequel le processus de décapage est réalisé et un contenant de liquide inférieur dans lequel une solution contenant de l'urée est collectée après le processus de décapage,
dans lequel l'évaporateur à vide comprend un récipient agencé pour retirer de l'eau d'une solution aqueuse d'urée pour la production d'une solution concentrée ou d'une fusion d'urée, ledit récipient comprenant un contenant de liquide inférieur où la solution concentrée ou la fusion d'urée est collectée ; le procédé comportant la fourniture :
a) d'écrans perforés agencés horizontalement dans la partie inférieure ;
dans laquelle l'option a) comprend la fourniture d'une pluralité de tamis perforés (20) agencés à une hauteur différente dans le récipient de liquide inférieur, la pluralité de tamis perforés comprenant des tamis discoïdes et des tamis annulaires empilés les uns au dessus des autres ;
dans lequel l'option a) comprend la fourniture de tamis agencés de sorte qu'au moins deux tamis qui sont placés l'un au-dessus de l'autre s'étendent sur différentes parties de section transversale d'une chambre interne (21) du contenant de liquide ;
dans lequel dans l'option a), un ou plusieurs tamis horizontaux sont intégrés à un déflecteur antitourbillon (16) monté à l'intérieur du contenant de liquide inférieur (11).
